Europäisches Patentamt

European Patent Office

Office européen des brevets

(11) Publication number: **0 238 772**
**A1**

## (12) EUROPEAN PATENT APPLICATION

(21) Application number: 86630137.7

(51) Int. Cl.⁴: **A61B 17/22**

(22) Date of filing: 04.09.86

(30) Priority: 09.09.85 IL 76344

(43) Date of publication of application:
**30.09.87 Bulletin 87/40**

(84) Designated Contracting States:
**AT BE CH DE FR GB IT LI LU NL SE**

(71) Applicant: **Ein-Gal, Moshe**
**3 Gilad Street**
**Ramat Hasharon(IL)**

(72) Inventor: **Ein-Gal, Moshe**
**3 Gilad Street**
**Ramat Hasharon(IL)**

(74) Representative: **Waxweiler, Jean et al**
**OFFICE DENNEMEYER S.à.r.l. P.O. Box 1502**
**L-1015 Luxembourg(LU)**

(54) **Apparatus for the disintegration of body calculi.**

(57) Non-invasive apparatus for the disintegration of body calculi comprising shock wave initiation apparatus (10 & 12), shock wave focusing apparatus the shape of which is selected from the group consisting of (a) no more than half longitudinally of an ellipsoidal-like inner surface (4) and (b) the inner surfaces of a plurality of cylindrical tubes which are so aligned as to focus at a single point (34), first shock wave liquid transmission apparatus (20) for transmitting the shock waves (32) from said initiation apparatus to a second shock wave liquid transmission apparatus (28), and shock wave liquid transmission apparatus for transmitting the shock aves from the first shock wave liquid transmission apparatus to a patient (30).

FIG 1

## FIELD OF THE INVENTION

The present invention relates to apparatus for the disintegration of body calculi and other brittle deposits in the body.

## BACKGROUND OF THE INVENTION

Calculi are stones which occur in certain hollow organs of the body or in ducts. They can cause obstruction, bleeding and when neglected, death. To this day, the most common method for removing such calculi is surgery. It is desirable to remove calculi without resort to surgery, and especially to be able to use a method which was non-invasive of the body. Similar remarks apply to the removal of calcium-containing deposits in arteries, as well as other calcified deposits and similarly brittle deposits elsewhere in the body. It may be noted that, while disintegrated kidney stones are flushed out of the body naturally, disintegrated arterial deposits, for example, would require special means for removal, such as "basketting" via cathetization.

Known non-invasive methods (described infra) have not achieved as widespread an application as could be desired, very largely because of the expense of installing the special equipment required.

U.S. Patent No. 4343301 to Indech provides a method of neural stimulation or local tissue destruction with a non-invasive technique wherein a pair of transducers direct high frequency sonic beams to a particular region in a patient's body, for the purpose of destroying, e.g., tumours and clots.

U.S. Patent No. 3958559 to Glenn et al similarly describes an ultrasonic transducer which destroys tumours by a non-invasive technique, in which sonic beams are directed towards a tumour region.

U.S. Patent No. 3735755 to Eggleton et al describes a non-invasive method and apparatus for destroying tissue using ultrasonic beams.

U.S. Patent No. 3735764 to Balev et al describes a method for removal of stones in the bladder wherein an electrode is introduced into the bladder and electrical impulses result in hydraulic shock waves which crush the stones.

U.S. Patent No. 3942531 to Hoff and Behrendt describes a non-invasive apparatus for breaking up concrements in the body, wherein shock waves are generated in a vessel which is either cylindrical or the greater part of an ellipsoid of which (as would appear from a drawing) perhaps an estimated one-quarter or so of the ellipsoidal volume at one end is removed, and the remaining approximately three-quarters is filled with water and placed against the human body. In the case of the ellipsoid, shock waves generated at a pair of electrodes near one focus of the ellipsoid are reflected off the longitudinal ellipsoidal surfaces and focused at what would be the other focus of the ellipsoid which is coincident with the kidney stone etc. which it is desired to be disintegrated.

U.S. Patent No. 4311147 to Hausler is somewhat akin to Hoff and Behrendt, in that it similarly describes a non-invasive apparatus for breaking up kidney stones and other calculi in the body, making use of shock waves, but in this case the latter are reflected from the inside surface of the bowl at one end of an ellipsoid, and focus at the ellipsoid focus at the other end, which is made coincident with the calculus to be disintegrated. The invention of this patent is more particularly characterized by a multiplicity of electrodes supported on an insulated carrier which, for the purpose of creating the desired shock waves, is positioned in the focus line at the reflector end of the apparatus, whereby spark gaps are constituted by successive pairs of electrodes in the array.

U.S. Patent 4,539,989 describes a instrument for destroying concretions within the body and includes a focusing chamber which is part of an ellipsoid of revolution, at one focus of which shock waves are generated by arc discharge. First and second water pads are provided, the first for coupling the shock waves to the body and the second for decoupling the shock waves from the body.

Existing methods which are based on principles of operation the same as or similar to the inventions of the three last-mentioned patents suffer certain disadvantages, more especially because they depend for success on being able to focus the shock waves precisely on the kidney stone etc. to be disintegrated, but details on how this is to be achieved are in fact lacking, at least in any meaningful sense. Thus, according to Hoff and Behrendt, the kidney stone is located "by means of an X-ray picture", while Hausler merely refers to a German Patent Application for details of how to adjust the focal point of the ellipsoid precisely on the stone etc. to be disintegrated. Nevertheless, it is well known to those skilled in the art that existing methods of this kind which are practiced at the present time require expensive apparatus for achieving the desired focusing; a special treatment installation is required for the patient, and the necessary equipment also includes integrated X-ray equipment. It is a great advantage of the present invention, that the need for such expensive apparatus is avoided, and use may be made of existing X-ray equipment. The apparatus of the invention is portable, and may be carried virtually anywhere.

Further disadvantages of such existing non-invasive methods for disintegrating kidney stones etc., result from the assumption which is made that the inner surface of an ellipsoid shell is the ideal configuration for bringing the shock waves to a precise focus. However, this assumption is not entirely true. The reason for this is that because of the dense medium i.e. water, which is used to fill the vessel, the shock waves do not behave in an exactly similar fashion as do light waves in air, for example, but rather they tend to diverge as they become progressively more concentrated near the desired focus. This means that the shock wave energy which ought to be concentrated at the desired point will to some extent be dispersed, and also that the focusing operation itself will not be as accurate as desired.

There is accordingly an apparent need to locate the object to be disintegrated so that the shock-wave energy can be directed accurately. Existing non-invasive methods for disintegrating kidney stones use a dedicated (dual) X-ray system as an integral part of the apparatus, which also comprises a special patient table (or bath) into which the shock wave generation and focusing assemblies are incorporated. Thus, such existing methods limit the use of shock wave technology, due to the high cost of the dedicated X-ray imaging system with the associated dedicated, specially designed table, room and/or bath. According to one aspect of the present invention, these disadvantages are in a substantial measure overcome, with the result that an imaging system and associated table which are available in a hospital or clinic, such as a fluoroscopic C-arm system, may be utilized. It will therefore be evident that more widespread availability of shock wave technology, as well as substantial economic advantages, will accrue from the use of the apparatus of the present invention.

## SUMMARY OF THE INVENTION

The present invention accordingly provides non-invasive apparatus for the disintegration of body calculi comprising:
shock wave initiation apparatus;
shock wave focusing apparatus the shape of which is selected from the group consisting of (a) no more than half longitudinally of an ellipsoidal-like inner surface and (b) the inner surfaces of a plurality of cylindrical tubes which are so aligned as to focus at a single point;
first shock wave liquid transmission apparatus for transmitting the shock waves from said initiation apparatus to a second shock wave liquid transmission apparatus; and

second shock wave liquid transmission apparatus for transmitting the shock waves from the first shock wave liquid transmission apparatus to a patient.

In one embodiment, the apparatus of the invention comprises:
shock wave initiation apparatus;
shock wave focusing apparatus the shape of which comprises no more than half longitudinally of an ellipsoidal-like inner surface;
first shock wave liquid transmission apparatus for transmitting the shock waves from said initiation apparatus to a second shock wave liquid transmission apparatus; and
second shock wave liquid transmission apparatus for transmitting the shock waves from the first shock wave liquid transmission apparatus to a patient.

In another embodiment, the apparatus of the invention comprises:
shock wave initiation apparatus;
shock wave focusing apparatus the shape of which comprises the inner surfaces of a plurality of cylindrical tubes which are so aligned as to focus at a single point;
first shock wave liquid transmission apparatus for transmitting the shock waves from said initiation apparatus to a second shock wave liquid transmission apparatus; and
second shock wave liquid transmission apparatus for transmitting the shock waves from the first shock wave liquid transmission apparatus to a patient.

The shock wave initiation apparatus may in general be either mechanical or electrical, but is preferably electrical. The shock wave initiation apparatus may comprise a plurality of shock wave initiation units, in which case the focusing means preferably comprises a respective focusing unit for each shock wave initiation unit. Alternatively the shock wave initiation apparatus may simply comprise a single unit. In a preferred embodiment of the invention, the shock wave initiation apparatus comprises at least one high tension electrode.

In a particularly preferred embodiment of the invention, the electrode is set in an insulated plug, and the latter is located in a non-curved wall or walls which together with the focusing means constitute a grounded, conductive vessel. It is believed that under these circumstances, current will flow from the electrode in two ways, firstly across the surface of the insulated plug to the nearest conducting surface, and secondly, if the water in the vessel is sufficiently conductive, through the water to the opposite and reflective face of the vessel. It is found that the water may be made sufficiently conductive for this purpose if e.g. 2% sodium chloride is added to distilled water. It will be appreciated that this two-fold system of conducting current from the electrode is a different principle from that utilized in known non-invasive methods and apparati generating shock waves, insofar as these make use of one pair or a plurality of pairs of immersed electrodes to create a spark therebetween, across a relatively short gap only, thus creating one or more spherical sources of shock waves. In the apparatus of the present invention the high concentration of current at the tip of the electrode causes a rapid evaporation of the liquid and the creation of a small bubble of non-conductive vapors. The high voltage across the bubble causes it to break down electrically and "explode", giving rise to shock waves emanating from the bubble into the liquid. The shape of the insulator supporting the electrode determines the angular distribution of the shock waves source; a hemispherical source, for example, is obtained by a planar insulator.

The desired properties of the insulating material are: high electrical resistivity and dielectric strength, ability to withstand the mechanical and thermal shocks associated with the "explosion", and it should also be inert chemically with respect to the liquid. Such a material is, for example, high-grade alumina ($Al_2O_3$).

When the embodiment of the focusing apparatus is that in which the shape comprises no more than half longitudinally of an ellipsoidal-like inner surface, the surface may be a modified ellipsoidal surface; for example, the surface at a plane section bisecting the apices of the ellipsoid-like shape may accord with the equation designated ($R_3$) (below).

The focusing means may comprise a single focusing unit. The latter may be, for example, the modified ellipsoidal surface as mentioned in the foregoing paragraph and described in detail infra. In one embodiment, such surface may be defined additionally in the following manner, namely, that when the longitudinal axis of the ellipsoidal-like surface is vertically disposed, a horizontal section therethrough has the approximate shape of either a half or a quarter of a circle, with the electrode located along the imaginary line which approximately passes perpendicularly to the plane of the horizontal section through the center of the circle.

In the apparatus of the invention, the initiation apparatus may comprise a plurality of high-tension electrodes and the focusing means may in this case comprise a respective focusing unit for the shock waves generated at each of the electrodes. In one embodiment of this aspect of the invention, the initiation apparatus comprises two high-tension electrodes and each focusing unit comprises an identical ellipsoidal-like surface, in particular the modified ellipsoidal surface mentioned above and described in more detail infra, of which each longitudinal axis is tilted in such manner that the respective sets of shock waves focus substantially at a common point, and the shape and disposition of each such surface is such that when a symmetrical vertical plane is taken through the intersection of the two such surfaces, then a horizontal plane taken through the uppermost point of contact of the pair of surfaces will take the form of a pair of semicircles touching at the common tangent, with an electrode located along the imaginary line which approximately passes perpendicularly to the horizontal plane through the center of the circle.

In a further embodiment of this aspect of the invention, the initiation apparatus comprises four high-tension electrodes and each focusing unit comprises an identical ellipsoidal-like surface, in particular the modified ellipsoidal surface mentioned above and described in more detail infra, and of which each longitudinal axis is tilted in such manner that the respective sets of shock waves focus substantially at a common point, and the shape and disposition of each such surface is such that when a symmetrical vertical plane is taken through the intersection of any two of the four opposing curved surfaces, then a horizontal plane taken through the uppermost point of contact of the four surfaces will take the form of a square where each of the surfaces constitutes approximately a quarter of a circle of which the centre is located at a corner of the square, an electrode being located along the imaginary line which approximately passes perpendicularly to the horizontal plane, through the centre of each of the four circles, and the common focus of the shock waves is located along the imaginary line which approximately passes perpendicularly to the horizontal plane at the center of the square.

It is preferred that each of the ellipsoidal-like surfaces previously described is additionally defined by a plane which passes through the centre but not the apices of the ellipsoid-like shape so as to symmetrically bisect the latter. This means that in this preferred form of the invention, the surface which will reflect and focus the shock waves comprises a portion of the surface of that half of an ellipsoid-like shape which has been produced by bisecting such shape equatorially.

In an alternative embodiment of the apparatus of the invention, the shock wave initiation apparatus is situated at the respective closed ends of the previously stated plurality of cylindrical tubes, each tube of which is open at one end, and the focusing apparatus is constituted by said tubes.

As regards another aspect of the invention, it is preferred that the first shock wave liquid transmission apparatus comprises at least one vessel with rigid longitudinal walls, of which at least one wall or a part thereof defines the shock wave focusing apparatus; that the second shock wave liquid transmission apparatus comprises at least one vessel with flexible longitudinal walls; and that where desired or necessary each of these vessels comprises means for filling them with and emptying them of liquid, as well as means for removing vapors generated during the "explosions", it being understood that the longitudinal walls are those walls which are to be disposed substantially perpendicularly to the body surface of a patient.

In one embodiment of this aspect of the invention, the first and second shock wave liquid transmission apparati are contiguous with no dividing wall therebetween. The second shock wave liquid transmission apparatus may be adapted so that the liquid therein makes direct contact with the body surface of the patient with no dividing wall therebetween, and it also comprises means for making a liquid-tight seal at the body surface. In the alternative, the second shock wave liquid transmission apparatus may comprise a flexible lateral wall adapted for direct contact with the body surface of a patient.

In a further embodiment of this aspect of the invention, the first shock wave liquid transmission apparatus comprises a flexible lateral wall adapted to abut against a flexible upper lateral wall in said second shock wave liquid transmission means, and the latter comprises also a flexible lower lateral wall adapted for direct contact with the body surface of a patient.

The present invention also comprises a method for the disintegration of body calculi and other brittle accretions in the body, by use of the apparatus described in the specification.

## BRIEF DESCRIPTION OF THE DRAWINGS

Fig. 1 illustrates in side-view section an embodiment of the apparatus of the invention comprising a single focusing unit;

Fig. 2 shows in plan the embodiment of Fig. 1 through plane 2-2 of Fig. 1;

Fig. 3 illustrates in side-view section a further embodiment of the apparatus of the invention comprising a single focusing unit;

Fig. 4 shows in plan the embodiment of Fig. 3 through plane 4-4 of Fig. 3;

Fig. 5 illustrates schematically in side-view section an embodiment of the apparatus of the invention comprising a pair of identical focusing units;

Fig. 6 shows in plan the embodiment of Fig. 5 through plane 6-6 of Fig. 5;

Fig. 7 shows schematically in plan an embodiment of the apparatus of the invention comprising four identical focusing units;

Fig. 8 illustrates pictorially an embodiment of the apparatus of the invention comprising a plurality of tubular focussing units;

Figs. 9A and 9B are side-view sectional representations of different embodiments of liquid transmission units forming part of the apparatus of the invention;

Figs. 10A and 10B are schematic side view sectional representations of the manner of locating a kidney stone in a patient prior to use of the apparatus of the invention;

Fig. 11 is a schematic side view of sectional representation taken by rotating at 90° in the horizontal plane the view of Figs. 10A or 10B, and showing additionally a liquid transmission unit which is a different embodiment from those shown in Figs. 9A and 9B;

Fig. 12 illustrates the determination of modified ellipsoidal reflectors; and

Fig. 13 is a side view sectional illustration of an alternative embodiment of focusing unit.

## DETAILED DESCRIPTION OF THE INVENTION

In the description of the drawings which follows, the same reference numerals have the same meaning throughout, unless the context requires otherwise.

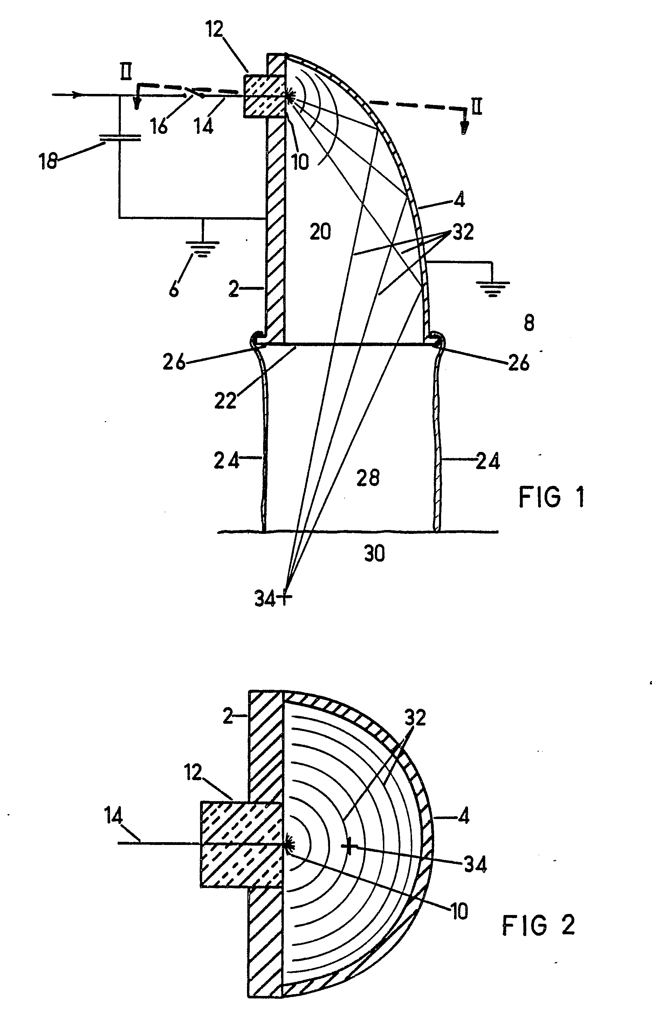

In Fig. 1, which illustrates in side-view section an embodiment of the apparatus of the invention comprising a single focusing unit, the latter comprises vertically orientated planar rigid wall (2) to which is sealably attached curved rigid wall (4) which has a shape approximating that of an equatorially bisected ellipsoidal-like surface such that the latter at a plane section bisecting the apices accords with the equation ($R_3$) (below).

Walls (2) and (4), which are preferably fabricated of stainless steel, are grounded at (6) and (8), respectively. Wall (2) contains as the preferred shock initiation device, an electrode (10), (preferably of tungsten, and, for example, of about 1 mm. diameter) set into insulating block (12), which is retained in (2) by means not shown. Electrode (10) is connected to a source of high tension electricity (not shown) by conductor (14), which may be (but need not be) also fabricated of tungsten, embodying switch (16) and grounded at (6) via a capacitor (18). Insulating block (12) is preferably cylindrically-shaped, but may alternatively have the shape of (for example) a rectangular prism; it should withstand a high electric field, high instantaneous thermal shock and mechanical shock, and is preferably constructed of alumina.

The interior (20) of the focusing unit is filled (by means not shown) with liquid, preferably 2% sodium chloride in distilled water. Alternatively, a non-electrolytic liquid may be used. The bottom end of this unit is (in this embodiment) closed by a thin flexible membrane (22), which also forms the top surface of a second vessel having flexible walls (24) which at their top end form a liquid-tight seal with the focusing unit by closing over flange (26) thereof.

The second vessel is also filled (by means not shown) with liquid (28), preferably water, and at its bottom end makes direct contact with the surface (30) of the body of a patient. In the embodiment illustrated, the second vessel has no bottom wall. The shock waves produced at (10) are shown - schematically (32); after bouncing off the interior curved surface of wall (4), these waves come to focus at a location (34), which will also be the location of the kidney stone to be disintegrated.

Fig. 2 shows a plan-like section through plane 2-2 (which is slightly tilted relative to the horizontal plane) of Fig. 1. It is seen that wall (4) in fact constitutes the surface of half of an equatorially bisected ellipsoid-like shape, which is of course otherwise as described with reference to Fig. 1.

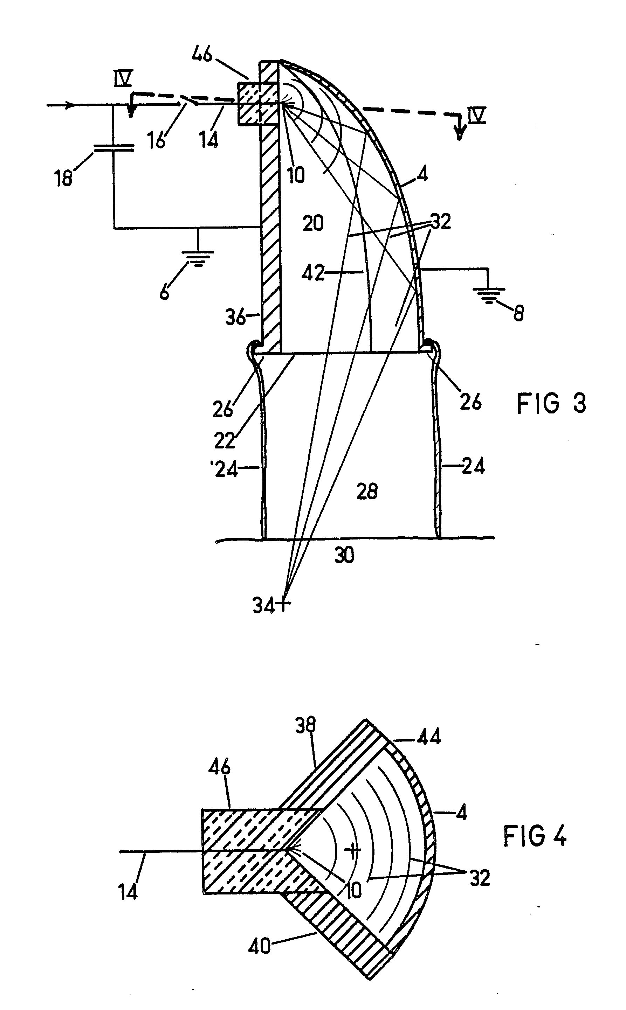

The next embodiment of the invention to be described will be best understood by considering Figs. 3 and 4 together. In this embodiment, the curved surface (4) represents one which approximates a quarter of an equatorially bisected ellipsoidal -like surface (rather than the half of such surface of the embodiment of Figs. 1 and 2). Consequently, in place of the planar surface (2) of Figs. 1 and 2, reference numeral (36) represents merely the thickness of the ridge of intersection of the two other vertical (planar) walls (38) and (40), of the focusing unit.

Curved line (42) (Fig. 3) represents in perspective, superimposed on an otherwise sectional representation, one extreme vertically disposed edge (44) (Fig. 4) of the curved surface. In this embodiment, the insulating block (46), corresponding with insulating block (12) of the previously described embodiment, is necessarily differently shaped from the latter; it may be in the shape of a cylinder with a portion having the shape of a 45° right cone removed from one end, or alternatively it may take the shape of a rectangular block with a 45° V-section prism having been removed from one end.

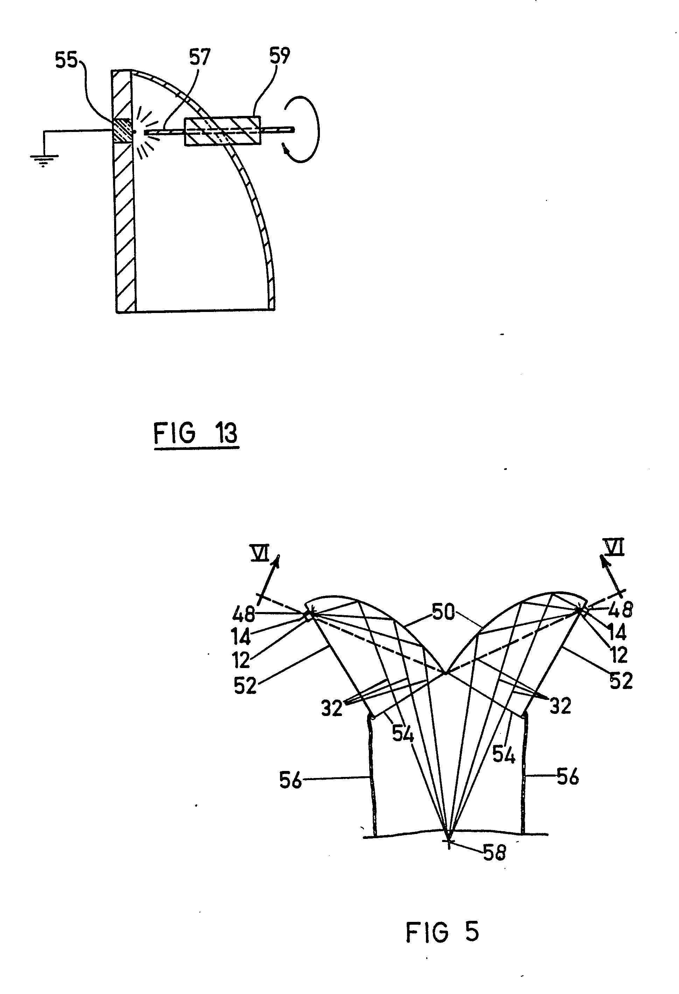

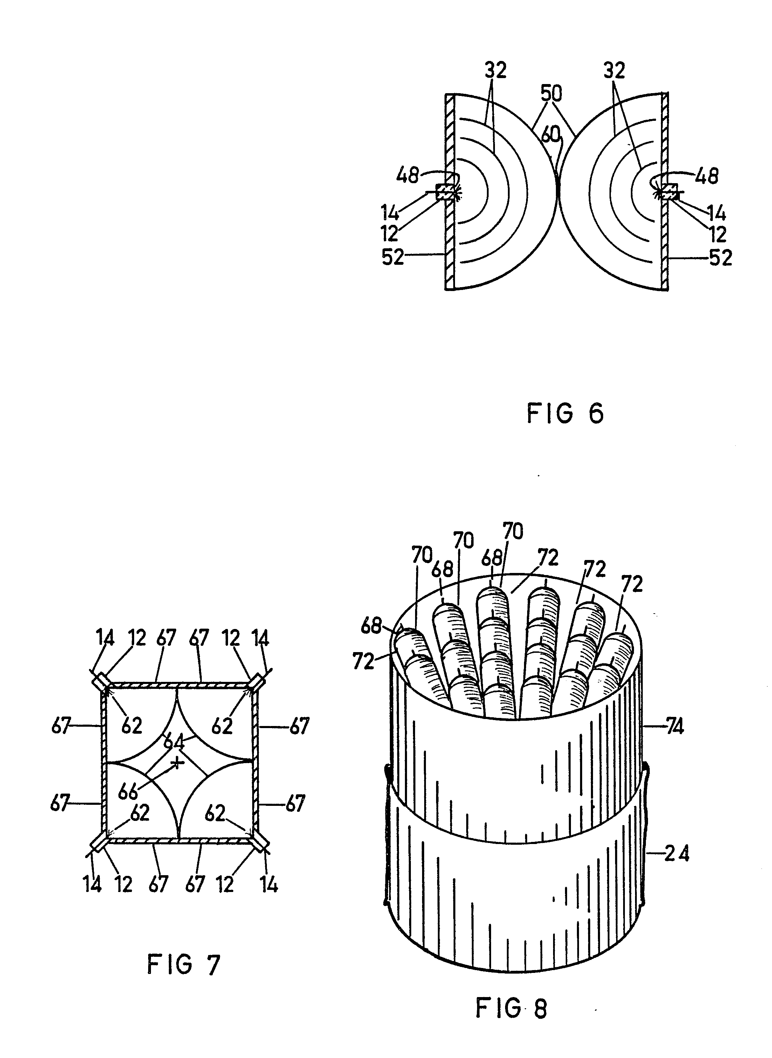

A further embodiment of the invention is shown in Fig. 5, which illustrates schematically in side-view section an apparatus comprising a pair of identical focussing units, and in Fig. 6, which illustrates in plan the embodiment of Fig. 5 through plane 6-6 of Fig. 5. Shock waves are initiated at two identical high-tension electrodes (48), and each respective focusing unit comprises an identical equatorially bisected ellipsoidal-like curved surface (50) as described above, which together with identical rigid planar walls (52) and identical flexible walls (54) comprise a first water-filled vessel for transmitting the generated shock waves; the latter are further transmitted to the body of a patient by a second vessel, which is defined by flexible walls (56).

As with the embodiments so far illustrated, the second vessel abuts directly onto the body surface. In this embodiment of the invention, the two curved surfaces (50) are so orientated with respect to each other, that the two streams of shock waves come to focus at point (58), where the kidney stone to be disintegrated is located.

It will be appreciated, especially by reference to Fig. 6, that the shape and disposition of each curved surface (50) is such that when a symmetrical vertical plane is taken through the intersection of the two curved surfaces, then a horizontal plane taken through the uppermost point of contact of the pair of surfaces will take the form of a pair of ellipse-like curves touching at the common tangent (60), with an electrode (48) located at the center of each curve.

In a further embodiment of the invention, shown schematically in plan in Fig. 7, shock waves are initiated at four identical high-tension electrodes (62), and each respective focusing unit comprises an identical one-quarter of an (equatorially bisected) ellipsoidal-like curved surface (64) as described above, and of which each longitudinal axis is tilted in such manner that the respective sets of shock waves focus substantially at the common point (66), which would be situated below the body surface of a patient and at the location of the kidney stone which it is desired to disintegrate.

It will be moreover observed from Fig. 7 that the shape and disposition of each curved surface (64) is such, that when a symmetrical vertical plane is taken through the intersection of any two of the four opposing curved surfaces, then a horizontal plane taken through the uppermost point of contact of the four surfaces will take the form of a square where each curved surface (64) constitutes approximately a quarter of a circle of which the centre is located at a corner of the square, an electrode (62) being located at each said center and the common focus (66) of the shock waves is located at the centre of the square, but of course well below the plane of this two-dimensional representation. The sides (67) of the square are of course formed by the rigid planar walls of each of the four vessels which embodies curved surface (64).

Reference is now made to Fig. 13, which illustrates an alternative embodiment of focusing unit having an overall configuration similar to that of the apparatus of Fig. 1. Here, however, the arc is generated between a grounded electrode 55 and an advancable electrode 57, which is consumed during operation and is mounted in an insulative rotatable feed mounting 59. This arrangement permits relatively long term use of the apparatus without stopping treatment to replace electrodes, as is necessary in other embodiments and in the prior art.

Fig. 8 illustrates an embodiment of the invention comprising a plurality of shock wave initiation units (68) each of which is situated at the respective closed ends (70) of a plurality of tubes (72) which are open at one end, and the focusing means is constituted by said tubes which are orientated in such manner as to direct the shock waves to substantially a common focus (not shown). In use, tubes (72) are filled with water and are supported in container (74); they are collectively equivalent to the first vessel in the embodiments which have been described above, but in this embodiment the second vessel with flexible walls is fitted over the end of container (74).

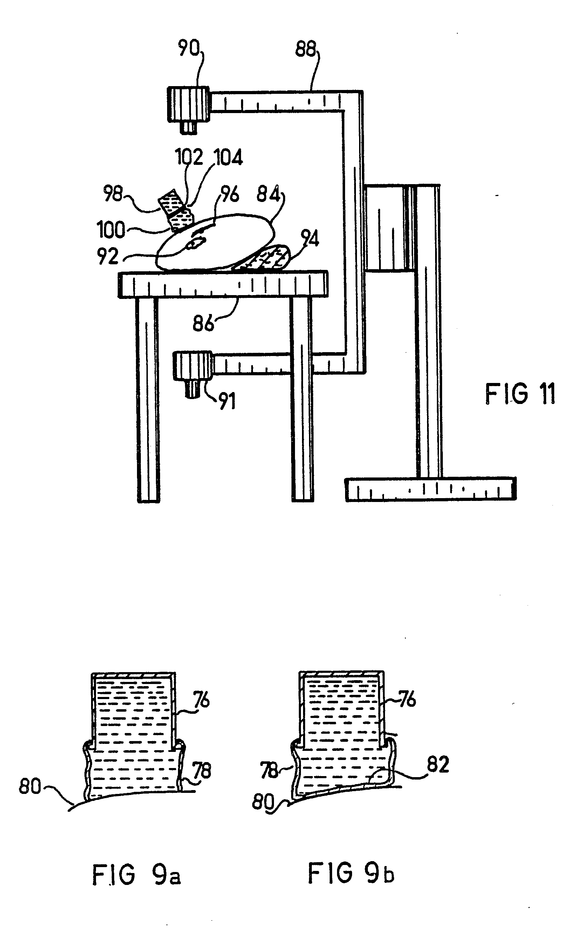

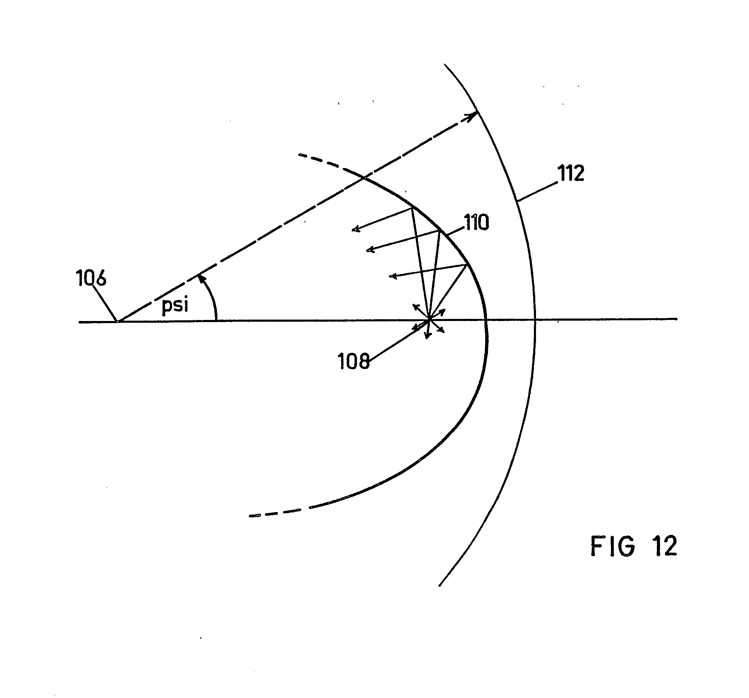

Fig. 9A shows in schematic side-view section a particular embodiment of the liquid transmission device which constitutes part of the apparatus of the invention. Rigid-walled vessel (76) is open at its lower end, on which is fastened the flexible-walled vessel (78); the latter abuts onto the surface (80) of the body of a patient with no intervening membrane. Fig. 9B shows a different embodiment of the liquid transmission device, characterized by the fact that in this case the flexible-walled vessel is closed at its lower end (82). In yet a different embodiment of the liquid transmission device (see for example Fig. 11, infra), the rigid-walled vessel may be closed at its lower end, and the flexible-walled vessel may be closed at its top end, by thin flexible walls which permit the transmission of shock waves from the former to the latter vessel.

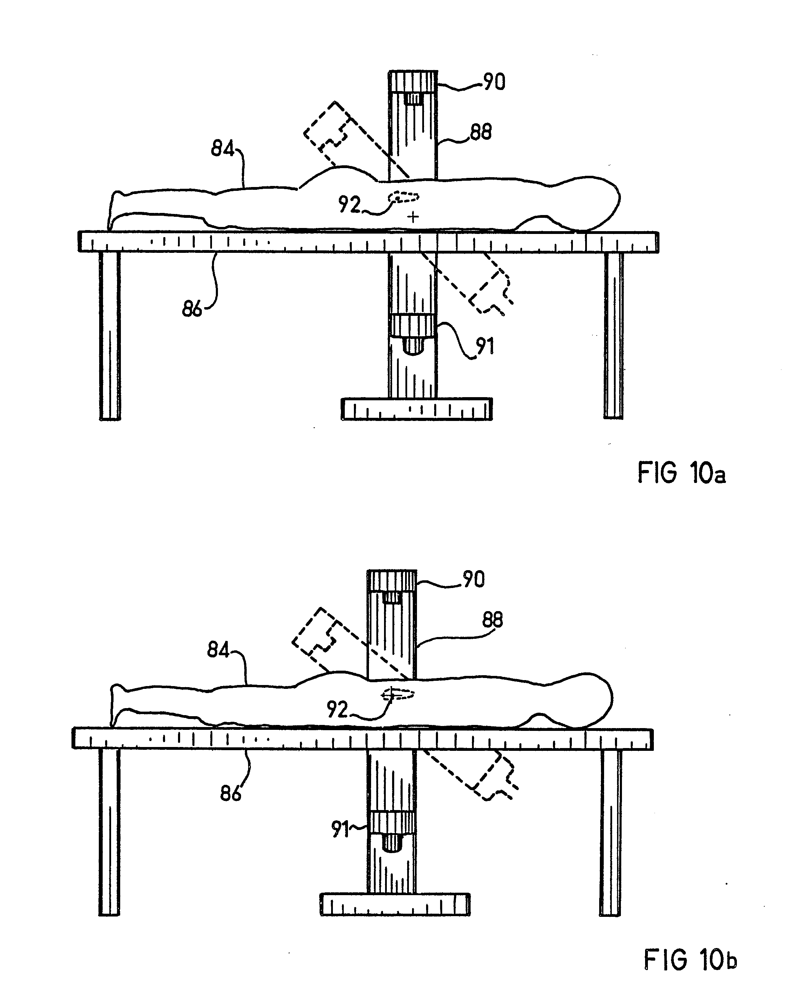

Figs. 10A and 10B are schematic side view sectional representation of the manner of locating a kidney stone in a patient prior to use of the apparatus of the invention. It is of course to be understood in the description which follows, that such terms as "vertical viewing tube" have the alternative meaning of "other analogous sighting means which perform the same function".

Firstly, it may be noted that the latter is in a fixed position, so that the location of the focus of the ellipsoid-like shock wave reflective surface will be precisely known. Patient (84) lies face downwards on table (86), which (by means not shown) may be moved vertically and/or raised or lowered as necessary - having regard also to the flexible-walled part of the apparatus of the invention which abuts onto the patient's body -in order that the focus of the reflective surface may be made precisely coincident with the kidney stone. At the top of C-arm (88) there is located X-ray source (90), and at the bottom an X-ray detection device (91) such as a fluoroscope.

By rotating the C-arm in a clockwise or counterclockwise direction, "sightings" of the kidney-stone (92) can be obtained. Thus for example when items (90) and (91) are in a vertical orientation, a vertical viewing tube will show the kidney stone, and the patient is moved as necessary so that the kidney stone is lined up with the known location of the focus of the reflective surface; at this point the focus of the surface is located somewhere on the imaginary vertical line through the stone. Next, the C-arm is rotated to, for example, an angle of 45° and the kidney stone is located through a viewing tube at this angle; the patient is now moved vertically until the view of the stone through the 45° viewing tube again is seen to coincide with the known location of the focus of the reflective surface. At this point, the stone has been made coincident with the focus of the reflective surface, and can be disintegrated with the apparatus of the invention. The broken lines show how the C-arm is rotated to a different position from the vertical. It should be noted that in general terms, in accordance with the invention, in the process of making coincident the object to be disintegrated and the focus of the shock wave reflector, a relative motion between the instrument and the patient is obtained by moving the patient, or the apparatus, or a combination of both. Moreover, a real-time tracking of the location of the target may incorporate visualization thereof, and changing the relative position of the patient and the shock wave apparatus accordingly.

In order to minimize X-radiation dosage, the location of the object to be disintegrated may be checked by the X-ray unit, say every 60 or 120 seconds. In between, a television camera or other sensor may provide an indication of the patient's movement. If movement is detected, the X-ray locating procedure is repeated.

As is shown more clearly in Fig. 11, the body of the patient (84) is in fact tilted with respect to the horizontal plane, and for this purpose, rests on pad (94); the spine is indicated at (96). Fig. 11 is a schematic side view sectional representation taken by rotating at 90° in the horizontal plane the view of Figs. 10A or 10B. As previously indicated, Fig. 11 shows a liquid transmission device which differs from those shown in Figs. 9A and 9B, in that the rigid-walled vessel (98) is closed at its lower end, and the flexible-walled vessel (100) is closed at its top end, by thin flexible walls (102) and (104), which permit the transmission of shock waves from the former to the latter vessel.

Since the present invention is concerned inter alia with the utilization of ellipsoidal-like surfaces, it will be relevant at this point to discuss the properties of such surfaces. Ellipsoidal reflectors have the following geometrical property: a straight line emanating from one focus is reflected at the wall of the ellipsoid and passes through the other focus. The total distance of such straight line from one focus to the wall, plus its straight line reflection from the wall to the other focus is the same for all such lines.

When using an ellipsoidal reflector for focusing shock waves (as in U.S. Patent No. 3942531), the source of the shock waves is placed in one focus of a partial ellipsoid and the "target" is placed in the other focus. The shock waves, however, do not propagate linearly in the target zone and tend to be deflected away from the ideal focus, causing the shock wave energy to be spread over a finite volume in the vicinity of the ideal focal point.

The present invention in a particular embodiment overcomes this difficulty by modifying the reflector curve to counter-deflect the waves and compensate for the natural deflection. The degree of compensatory modification necessary will depend on the intensity of the shock waves and the properties of the medium in which they propagate; the higher the intensity and the associated natural deflection, the larger the deviation of the reflector curve from that of an ellipsoid. The dependence of the modification on the physical conditions leads to a family of curves where a parameter determines the particular curve for a given set of physical conditions (see Fig. 12). Such a family of polar coordinated curves $R(D, phi)$ is given, for example by the expression:

$R(D, phi) = [A(1-c^2) - D + Dcos(phi)]/[1-c.cos(phi)] ...(R_1)$

where $c = P/A$, 2P is the distance from the origin (106) to the source (108) (Fig. 12) and A - P is the shortest distance from the source (108) to the reflector (110). $R( )$ and phi are the polar coordinates, the target focus is coincident with the origin (106), and the parameter D specifies the amount of deviation from an ellipsoid (for $D = 0$ the curve is an ellipsoid). A method for generating families of curves is now presented. The method assumes the knowledge of a desired converging wave-front (112) $[W(psi)]$ given in polar coordinates as before and subject to the scaling condition $W(0) = 2A$. The Cartesian coordinates x, y of the associated reflector curve are then given by

$x = 2d + R'cos(psi'); y = R'sin(psi') ... (R_2)$

where $R' = (g^2 -1)/[g - cos(psi')]$;

$g = Wsin(psi)/[Wsin(gamma) + 2Psin(psi')]$;

$psi' = psi - gamma$;

$2d = -Wsin(gamma)$; and gamma is given by

$tan(gamma) = [dW/d(psi)]/W$.

It is to be noted that when the converging wavefront is spherical, then $W(psi) = 2A$ (as in the case of very weak waves) and gamma = d = 0, g = A/P, so that the reflector curve is again an ellipsoid. The family of reflector curves is then associated with the parameterized family of wavefront functions $W(D, psi)$. For example, if

$W(D, psi) = (2A - D)\{1-[D/(2A - D)]^2\}/[1 - Dcos(psi)/2A -D)]$

then the parameter D represents the amount of deflection and the corresponding deviation of the reflector curve from that of an ellipsoid. Note again that $D = 0$ represents a spherical wavefront and an associated ellipsoidal reflector.

The parameters A and P determine the size and shape of the reflector independently of the deviation parameter D. For example, in the application of such a reflector for the purpose of kidney stone disintegration via shock waves, A is between 10 and 18 cm. and P is between 9 and 15 cm. The general expression for the modified ellipsoidal reflector curve [examples of which are $(R_1)$ and $(R_2)$] is $R(D', phi)$ in polar coordinates:

$R(D', phi) = M(D', phi).R(phi) ... (R_3)$

where $D' = D/A$, $R(phi)$ is a perfect ellipsoid and $M(D', phi)$ is the modifying function.

While a number of specific embodiments of the invention have been described, it will be appreciated by those skilled in the art that many variations and modifications of the described apparatus may be made. The invention is accordingly not to be construed as limited to the specific embodiments particularly described, but on the contrary, its scope is restricted only by the claims which follow.

**Claims**

1. Non-invasive apparatus for the disintegration of body calculi and other brittle accretions comprising:

shock wave initiation means;

shock wave focusing means the shape of which is selected from the group consisting of (a) no more than half longitudinally of an ellipsoidal-like inner surface and (b) the inner surfaces of a plurality of cylindrical tubes which are so aligned as to focus at a single point;

first shock wave liquid transmission means for transmitting the shock waves from said initiation means to a second shock wave liquid transmission means; and

second shock wave liquid transmission means for transmitting the shock waves from the first shock wave liquid transmission means to a patient.

2. Non-invasive apparatus for the disintegration of body calculi and other brittle accretions comprising:

shock wave initiation means:

shock wave focusing means the shape of which comprises no more than half longitudinally of an ellipsoidal-like inner surface;

first shock wave liquid transmission means for transmitting the shock waves from said initiation means to a second shock wave liquid transmission means; and

second shock wave liquid transmission means for transmitting the shock waves from the first shock wave liquid transmission means to a patient.

3. Apparatus according to claim 2 wherein the initiation means comprises a plurality of shock wave initiation units and the focusing means comprises a respective focusing unit for each shock wave initiation unit.

4. Apparatus according to claim 1 wherein the shock wave initiation means comprises at least one high tension electrode.

5. Apparatus according to claim 4 wherein the electrode is set in an insulated plug, and the latter is located in a non-curved wall or walls which together with the focusing means constitute a grounded, conductive vessel.

6. Apparatus according to claim 5 wherein the ellipsoidal-like reflector surface is defined by a family of polar coordinated curves R(D, phi) in accordance with the expression:

$$R(D, phi) = [A(1-c^2) - D + Dcos(phi)]/[1-c.cos(phi)] ...(R_1)$$

where R(D) and phi are the polar coordinates, c = P/A, 2P is the distance from the target to the source of shock waves, A - P is the shortest distance from the said source to the said reflector surface and the parameter D represents the amount of deflection and the corresponding deviation of the reflector curve from that of an ellipsoid.

7. Apparatus according to claim 5 wherein the ellipsoidal-like reflector surface is defined by the Cartesian coordinates x, y, wherein

$$x = 2d + R'cos(psi'); y = R'sin(psi') ... (R_2)$$

where psi is the angle made by the shock wavefront W with the x-axis;

$R' = (g^2 -1)/[g - cos(psi')]$;

$g = Wsin(psi)/[Wsin(gamma) + 2Psin(psi')]$;

2P is the distance from the target to the source of shock waves;

psi' = psi - gamma;

2d = -Wsin(gamma); and gamma is given by

$tan(gamma) = [dW/d(psi)] /W$.

8. Apparatus according to claim 5 wherein the ellipsoidal-like reflector surface R(D', phi) in polar coordinates is defined in accordance with the expression

$$R(D', phi) = M(D', phi).R(phi) ... (R_3)$$

where D' = D/A, R(phi) is a perfect ellipsoid and M(D', phi) is the modifying function, the parameter D represents the amount of deflection and the corresponding deviation of the reflector curve from that of an ellipsoid, and the parameter A determines the size and shape of the reflector surface independently of the parameter D.

9. Apparatus according to claim 6 wherein A is between about 10 and about 18 cm. and P is between about 9 and about 15 cm.

10. Apparatus according to claim 4 wherein the focusing means comprises a single focusing unit.

11. Apparatus according to claim 10 wherein the single focusing unit is a surface as defined such that when the longitudinal axis thereof is vertically disposed, a horizontal section therethrough has an approximately semicircular shape, with the electrode located along the imaginary line which approximately passes perpendicularly to the plane of the horizontal section, through the center of the circle.

12. Apparatus according to claim 10 wherein the single focusing unit is a surface as defined by a family of polar coordinated curves R(D, phi) in accordance with the expression:

$$R(D, phi) = [A(1-c^2) - D + Dcos(phi)]/[1-c.cos(phi)] ...(R_1)$$

where R(D) and phi are the polar coordinates, c = P/A, 2P is the distance from the target to the source of shock waves, A - P is the shortest distance from the said source to the said reflector surface and the parameter D represents the amount of deflection and the corresponding deviation of the reflector curve from that of an ellipsoid and such that when the longitudinal axis thereof is vertically disposed, a horizontal section therethrough has the approximate shape of a quarter of a circle, with the electrode located along the imaginary line which approximately passes perpendicularly to the plane of the horizontal section, through the centre of the circle.

13. Apparatus according to claim 2 wherein the initiation means comprises a plurality of high-tension electrodes and the focusing means comprises a respective focusing unit for the shock waves generated at each of the electrodes.

14. Apparatus according to claim 13 wherein the initiation means comprises two high-tension electrodes and two focusing units associated therewith, each focusing unit comprises an identical surface as defined in any of claims 8 to 11, and of which each longitudinal axis is tilted in such manner that the respective sets of shock waves focus substantially at a common point, and the shape and disposition of each such surface is such that when a symmetrical vertical plane is taken through the intersection of the two identical surfaces, then a horizontal plane taken through the uppermost point of contact of the pair of surfaces will take the form of a pair of semicircles touching at the common tangent, with an electrode located at the centre of each circle.

15. Apparatus according to claim 13 wherein the initiation means comprises four high-tension electrodes and each focusing unit comprises an identical surface as defined by a family of polar coordinated curves R(D, phi) in accordance with the expression:

$$R(D, phi) = [A(1-c^2) - D + Dcos(phi)]/[1-c.cos(phi)] \quad ...(R_1)$$

where R(D) and phi are the polar coordinates, c = P/A, 2P is the distance from the target to the source of shock waves, A - P is the shortest distance from the said source to the said reflector surface and the parameter D represents the amout of deflection and the corresponding deviation of the reflector curve from that of an ellipsoid and of which each longitudinal axis is tilted in such manner that the respective sets of shock waves focus substantially at a common point, and the shape and disposition of each surface is such that when a symmetrical vertical plane is taken through the intersection of any two of the four opposing surfaces, then a horizontal plane taken through the uppermost point of contact of the four surfaces will take the form of square where each surface constitutes approximately a quarter of a circle of which the centre is located at a corner of the square, an electrode being located along the imaginary line which approximately passes perpendicularly to the horizontal plane, through the centre of each of the four circles, and the common focus of the shock waves is located along the imaginary line which approximately passes perpendicularly to the horizontal plane at the center of the square.

16. Apparatus according to claim 6 wherein the ellipsoidal-like surface is additionally defined by a plane which passes through the centre but not the apices of the ellipsoid-like shape so as to symmetrically bisect the latter.

17. Apparatus according to claim 5, wherein the insulated plug is constituted of material which possesses high electrical resistivity and dielectric strength, and ability to withstand the mechanical and thermal shocks associated with an explosive electrical breakdown of the bubble of non-conductive vapors which forms at the tip of the electrode, and which is chemically inert with respect to the liquid with which it is in contact.

18. Apparatus according to claim 2 wherein:
said first shock wave liquid transmission means comprises at least one vessel with rigid longitudinal walls, of which at least one wall or a part thereof defines said shock wave focusing means;
said second shock wave liquid transmission means comprises at least one vessel with flexible longitudinal walls; and
where desired or necessary each of said vessels comprises means for filling them with and emptying them of liquid as well as means for removing generated vapors;
said longitudinal walls being defined as those walls which are to be disposed substantially perpendicularly to the body surface of a patient.

19. Apparatus according to claim 18 wherein said first shock wave liquid transmission means and said second shock wave liquid transmission means are contiguous with no dividing wall therebetween.

20. Apparatus according to claim 19 wherein said second shock wave liquid transmission means is adapted so that the liquid therein makes direct contact with the body surface of the patient with no dividing wall therebetween, and the said second means comprises also means for making a liquid-tight seal at the body surface.

21. Apparatus according to claim 19 wherein said second shock wave liquid transmission means comprises a flexible lateral wall adapted for direct contact with the body surface of a patient.

22. Apparatus according to claim 20 wherein said first shock wave liquid transmission means comprises a flexible lateral wall adapted to abut against a flexible upper lateral wall in said second shock wave liquid transmission means, and the latter comprises also a flexible lower lateral wall adapted for direct contact with the body surface of a patient.

23. Non-invasive apparatus for the disintegration of body calculi and other brittle accretions comprising:
shock wave initiation means;
shock wave focusing means the shape of which comprises the inner surfaces of a plurality of cylindrical tubes which are so aligned as to focus at a single point;
first shock wave liquid transmission means for transmitting the shock waves from said initiation means to a second shock wave liquid transmission means; and
second shock wave liquid transmission means for transmitting the shock waves from the first shock wave liquid transmission means to a patient.

FIG 1

FIG 2

FIG 3

FIG 4

FIG 13

FIG 5

FIG 6

FIG 7

FIG 8

FIG 11

FIG 9a          FIG 9b

FIG 10a

FIG 10b

FIG 12

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X,D | EP-A-0 081 639 (DORNIER) | | A 61 B 17/22 |
| | * Figures; abstract; claim 1 * | 1,2 | |
| Y | | 3-5, 10,11 | |
| | --- | | |
| Y | DE-A-2 744 823 (ENDRESS) | | |
| | * Figures 4-6; page 13, lines 7-14, 18-28 * | 3-5, 10,11 | |
| A | | 13,14 | |
| | --- | | |
| A | EP-A-0 133 946 (SIEMENS AG) | | |
| | * Figures 2-4; page 7, lines 28-35; page 10, lines 8-26 * | 18,19, 21,23 | |
| | --- | | |
| A | EP-A-0 090 138 (DORNIER SYSTEMS) | | |
| | * Figure 1; abstract; page 3, line 34 - page 4, line 10 * | 18 | TECHNICAL FIELDS SEARCHED (Int. Cl.4) |
| | --- | | A 61 B |
| A | DE-A-3 220 751 (SCHULLER) | | G 10 K |
| | * Figures; page 6, lines 16-20 * | 20 | |
| | --- | | |
| A | DE-A-3 312 014 (EISENMENGER) | | |
| | * Figure 4; page 18, lines 3-8 * | | |
| | -------------------- | | |

The present search report has been drawn up for all claims

| Place of search | Date of completion of the search | Examiner |
|---|---|---|
| The Hague | 14-05-1987 | SEDY |

European Patent
Office

## CLAIMS INCURRING FEES

The present European patent application comprised at the time of filing more than ten claims.

☐ All claims fees have been paid within the prescribed time limit. The present European search report has been drawn up for all claims.

☐ Only part of the claims fees have been paid within the prescribed time limit. The present European search report has been drawn up for the first ten claims and for those claims for which claims fees have been paid,

namely claims:

☐ No claims fees have been paid within the prescribed time limit. The present European search report has been drawn up for the first ten claims.

## X  LACK OF UNITY OF INVENTION

The Search Division considers that the present European patent application does not comply with the requirement of unity of invention and relates to several inventions or groups of inventions,

namely:

```
1) Claims 1-17: Ellipsoidal-like shock wave focusing
               means

2) Claims 1,2,18-22: Shock wave liquid transmission
                     means

3) Claims 1,23: Plurality of cylindrical tubes as
                focusing means
```

☒ All further search fees have been paid within the fixed time limit. The present European search report has been drawn up for all claims.

☐ Only part of the further search fees have been paid within the fixed time limit. The present European search report has been drawn up for those parts of the European patent application which relate to the inventions in respect of which search fees have been paid,

namely claims:

☐ None of the further search fees has been paid within the fixed time limit. The present European search report has been drawn up for those parts of the European patent application which relate to the invention first mentioned in the claims,

namely claims: